# EUROPEAN PATENT APPLICATION

(11) **EP 3 556 410 A1**
(43) Date of publication of application: **23.10.2019**
(21) Application number: 17879877.3
(22) Date of filing: 11.12.2017
(51) Int. Cl.: A61M 1/36, A61M 25/00, A61M 25/14

(54) **CATHETER**

(30) Priority: 16.12.2016 JP 2016244565
(71) Applicant: Terumo Kabushiki Kaisha, Tokyo 151-0072 (JP)
(72) Inventor: YOKOYAMA, Kenji, Ashigarakami-gun Kanagawa 259-0151 (JP)
(74) Representative: Casalonga
(86) International application number: PCT/JP2017/044356
(87) International publication number: WO 2018/110494

(57) **Abstract**

[Problem] To provide a catheter that can disperse blood flowing out of it and soften the collision of the blood with the blood vessel wall.

[Means for Resolution] A catheter 60 includes a blood feeding lumen 61 extending in an axial direction, a blood feeding side hole 63 communicating with a distal end of the blood feeding lumen, and a rectification part 70 that is disposed in the blood feeding lumen at a position facing the blood feeding side hole of the blood feeding lumen and rises in a direction intersecting with the axial direction.

## Description

### Technical Field

The present invention relates to a catheter.

### Background Art

Conventionally, in order to provide cardiopulmonary resuscitation, circulatory support, and respiratory support in emergency treatment, treatment with percutaneous cardiopulmonary support (PCPS) has been performed. Percutaneous cardiopulmonary support is a method of temporarily assisting and covering cardiopulmonary function using an extracorporeal circulator. In addition, the extracorporeal circulator is also used in open-heart surgery.

The extracorporeal circulator has an extracorporeal circulation circuit including a centrifugal pump, an oxygenator, a blood removal path, a blood feed path, and the like, and is configured to perform gas exchange on removed blood and feed the blood to the blood feed path.

In connection with this, Patent Literature 1 below, for example, describes a circulation circuit in an extracorporeal circulator.

### Citation List

### Patent Literature

PTL 1: International Publication No. WO2007/123156

### Summary of Invention

### Technical Problem

In such circulation circuits, a blood feed catheter is used to send blood to a desired position in the living body. In such a blood feed catheter, the inner diameter of an outlet port is generally smaller than the inner diameter of the blood vessel, so that blood flowing out from the outlet port may be fast and strongly collide with the blood vessel wall.

In order to solve the above-mentioned problems, it is an object of the present invention to provide a catheter that can disperse blood flowing out of it and soften the collision of the blood with the blood vessel wall.

### Solution to Problem

A catheter achieving the above-described object is a catheter extending in an axial direction and passing blood, and includes a blood feeding lumen extending in the axial direction; a blood feeding side hole communicating with a distal end of the blood feeding lumen; and a rectification part that is disposed at a position facing the blood feeding side hole of the blood feeding lumen and rises in a direction intersecting with the axial direction.

### Advantageous Effects of Invention

In the catheter configured as described above, the rectification part is provided at a position facing the blood feeding side hole, so that blood passing through the blood feeding lumen can collide with the rectification part and thus the blood flowing out from the catheter can be dispersed. Therefore, according to the catheter configured as described above, it is possible to soften the blood collision with the blood vessel wall.

### Brief Description of Drawings

Fig. 1 is a system diagram illustrating an example of an extracorporeal circulator to which a catheter according to an embodiment of the present invention is applied.
Fig. 2 is a top view illustrating a catheter assembly before an insertion of a dilator into the catheter according to the embodiment.
Fig. 3 is a cross-sectional side view illustrating the catheter.
Fig. 4 is a top view of the catheter assembly after the insertion of the dilator into the catheter.
Fig. 5 is a perspective view for explaining a configuration of a rectification part.
Fig. 6 illustrates drawings for explaining a configuration of the rectification part, in which Fig. 6A is a top view, and Fig. 6B is a cross-sectional view taken along a line 6B-6B in Fig. 6A.
Fig. 7 is a schematic cross-sectional view illustrating a distal end side of a dilator assembly after the insertion of the dilator into the catheter.
Fig. 8 is a drawing illustrating a distal tip.
Fig. 9 is a drawing corresponding to Fig.5 illustrating a catheter according to a comparative example.
Fig. 10 illustrates pictures for explaining an effect of the catheter according to the present embodiment, in which Fig. 10A shows a state of water flowing out from a blood feeding side hole of the catheter according to the embodiment viewed from a side, and Fig. 10B shows a state of water flowing out from a blood feeding side hole of the catheter according to the comparative example viewed from the side.
Fig. 11 illustrates pictures for explaining an effect of the catheter according to the present embodiment, in which Fig. 11A shows a state of water flowing out from the blood feeding side hole of the catheter according to the embodiment viewed from a top, and Fig. 11B shows a state of water flowing out from the blood feeding side hole of the catheter according to the comparative example viewed from the top.
Fig 12 is a graph for explaining the effect of the catheter according to the present embodiment.
Fig. 13 is a drawing corresponding to Fig. 5, illustrating a catheter according to a modified example 1.
Fig. 14 is a drawing corresponding to Fig. 5, illustrating a catheter according to a modified example 2.
Fig. 15 is a drawing corresponding to Fig. 5, illustrating a catheter according to a modified example 3.
Fig. 16 is a drawing corresponding to Fig. 5, illustrating a catheter according to a modified example 4.
Fig. 17 is a drawing illustrating a catheter according to a modified example 5.

### Description of Embodiments

Referring now to the attached drawings, embodiments of the present invention will be described. Note that the following description is not intended to limit the technical scope or significance of terms described in Claims. Dimensional ratios of the drawings are exaggerated for the convenience of description and may be different from actual ratios.

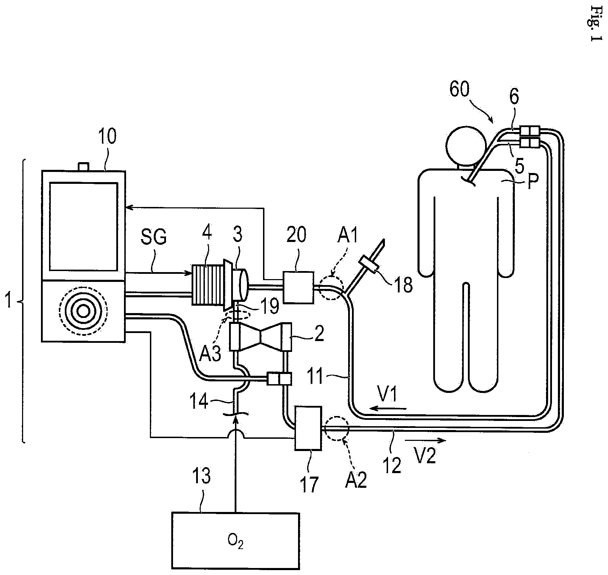

FIG. 1 is a system diagram illustrating an example of an extracorporeal circulator in which the catheter according to the embodiment of the present invention is used and which is used, when the heart of a patient is weakened, for percutaneous cardiopulmonary support (PCPS) to temporarily assist and cover the functions of the heart and the lungs until the heart function is recovered.

According to an extracorporeal circulator 1, a Veno-Venous (VV) method can be performed. The VV method includes activating a pump to remove blood from patient's vein (vena cava), performing gas exchange of blood by an oxygenator for oxygeneration of blood, and then returning the blood back to the patient's vein (vena cava) again. The extracorporeal circulator 1 is an apparatus for assisting the heart and the lungs. Hereinafter, a method of removing blood from a patient, performing a predetermined treatment extracorporeally, and then feeding the blood back to the patient's body is referred to as "extracorporeal circulation".

As illustrated in Fig. 1, the extracorporeal circulator 1 includes a circulation circuit for circulating blood. The circulation circuit includes an oxygenator 2, a centrifugal pump 3, a drive motor 4, which is driving means for driving the centrifugal pump 3, a catheter 60, and a controller 10 as a control unit.

The catheter 60 includes a blood removing tube 5 in which a blood removing lumen that functions as a blood removal path is formed and a blood feeding tube 6 in which a blood feeding lumen that functions as a blood feed path is formed.

The catheter 60 is inserted from an internal jugular vein at a neck, passes through a superior vena cava and a right atrium, and a distal end of the catheter 60 is indwelled in an inferior vena cava. A target of blood feed of the blood feeding lumen is the right atrium. A target of blood removal of the blood removing lumen is two locations; the superior vena cava and the inferior vena cava.

The blood removing tube 5 is connected to the centrifugal pump 3 via a blood removal tube (blood removal line) 11. The blood removal tube 11 is a pipe line for feeding blood.

When the drive motor 4 activates the centrifugal pump 3 upon reception of command SG from the controller 10, the centrifugal pump 3 can remove blood from the blood removal tube 11, pass the blood through the oxygenator 2, and then return the blood back to a patient P via a blood feed tube (blood feed line) 12.

The oxygenator 2 is positioned between the centrifugal pump 3 and the blood feed tube 12. The oxygenator 2 performs gas exchange (addition of oxygen and/or removal of carbon dioxide) for blood. The oxygenator 2 is, for example, an extracorporeal membrane oxygenator, and specifically, a hollow fiber extracorporeal membrane oxygenator is preferably used. Oxygen gas is fed from an oxygen gas supply unit 13 to the oxygenator 2 via a tube 14. The blood feed tube 12 is a pipe line that connects the oxygenator 2 and the blood feeding tube 6.

The blood removal tube 11 and the blood feed tube 12 may be, for example, pipe lines made of a flexible synthetic resin having a high transparency and a resiliently deformable property such as a vinyl chloride resin or a silicone rubber. In the blood removal tube 11, blood, which is a liquid, flows in a direction V1, and in the blood feed tube 12, the blood flows in a direction V2.

In the circulation circuit illustrated in Fig. 1, an ultrasound bubble detection sensor 20 is disposed in a middle of the blood removal tube 11. A fast clamp 17 is disposed in a middle of the blood feed tube 12.

When air bubbles enter into the circulation circuit due to an erroneous operation of a three-way stopcock 18 or breakage of the tube during the extracorporeal circulation, the ultrasound bubble detection sensor 20 detects the air bubbles entered therein. When the ultrasound bubble detection sensor 20 detects that air bubbles are present in blood fed into the blood removal tube 11, the ultrasound bubble detection sensor 20 sends a detection signal to the controller 10. The controller 10 notifies a warning by an alarm based on the detection signal, and lowers the number of rotation of the centrifugal pump 3 or stops the centrifugal pump 3. In addition, the controller 10 sends a command to the fast clamp 17, and the fast clamp 17 immediately blocks the blood feed tube 12. Accordingly, the air bubbles are prevented from being delivered to the body of the patient P. The controller 10 controls the operation of the extracorporeal circulator 1 to prevent the air bubbles from entering the body of the patient P.

The tube 11 (12, 19) of the circulation circuit of the extracorporeal circulator 1 is provided with a pressure sensor. The pressure sensor may be mounted all or any one of a mounting position A1 on the blood removal tube 11, a mounting position A2 on the blood feed tube 12 of the circulation circuit, and a mounting position A3 of a connecting tube 19 connecting the centrifugal pump 3 and the oxygenator 2. Accordingly, when extracorporeal circulation is performed for the patient P by the extracorporeal circulator 1, the pressure in the tube 11 (12, 19) can be measured by the pressure sensor. Note that the mounting position of the pressure sensor is not limited to the above-described mounting positions A1, A2, and A3, and may be any positions on the circulation circuit.

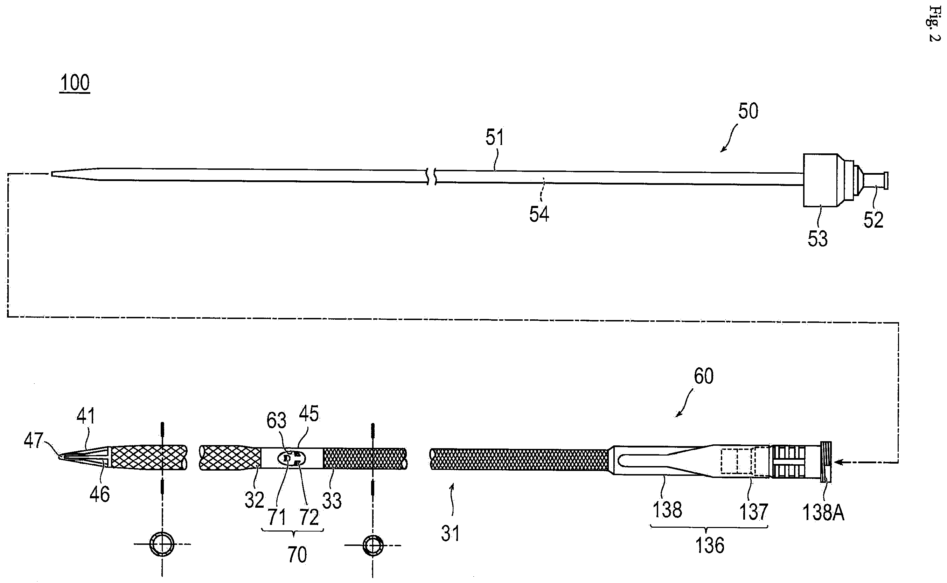

Referring next to Fig. 2 to Fig. 8, a catheter assembly 100 according to the embodiment of the present invention will be described. Fig. 2 to Fig. 8 are drawings for explaining a configuration of the catheter assembly 100 according to the embodiment.

The catheter assembly 100 according to the present embodiment includes the catheter 60 configured to pass blood and a dilator 50 to be inserted into the catheter 60 as illustrated in Fig. 2. The catheter 60 is used as the catheter 60 in Fig. 1.

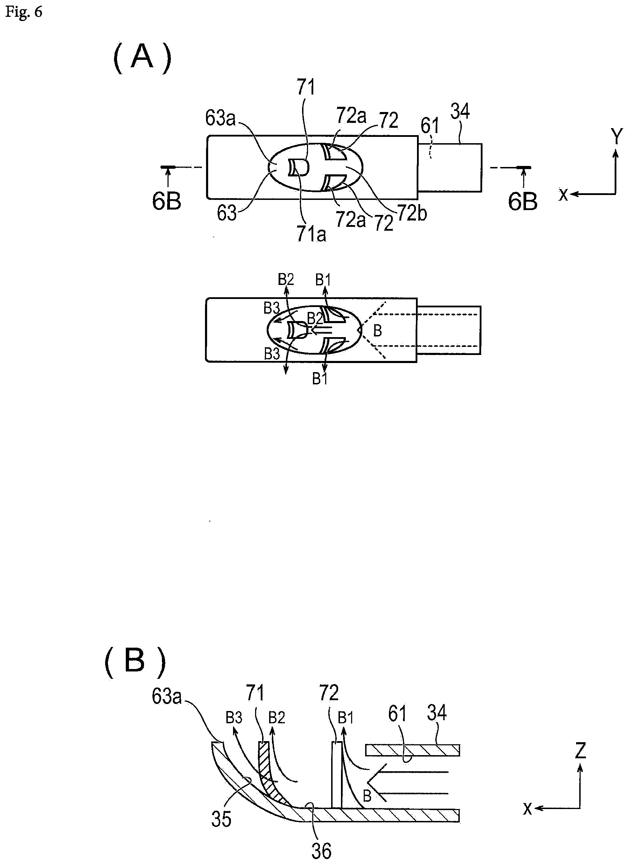

Note that, in this specification, a side to be inserted into the living body is referred to as "distal end" or "distal side", and a hand-side where an operator operates is referred to as "proximal end" or "proximal side". The distal end portion indicates a certain range including the distal end (distal-most end) and a periphery thereof, and the proximal portion indicates a certain range including a proximal end (proximal-most end) and a periphery thereof. An axial direction of the catheter 60 is referred to as "X direction", an up-down direction in Fig. 6A is referred to as "Y direction", and an up-down direction in Fig. 6B is referred to as "Z direction".

The catheter 60 according to the present embodiment is a so-called double-lumen catheter, and is capable of performing both blood feed and blood removal simultaneously.

As illustrated in Fig. 2 to Fig. 4, the catheter 60 includes a catheter tube 31, a connector 45 configured to connect a first tube 32 and a second tube 33 of the catheter tube 31, a rectification part 70 fixed to a distal end of a third tube 34 of the catheter tube 31, a distal tip 41 disposed at a distal end of the first tube 32, and a lock connector 136.

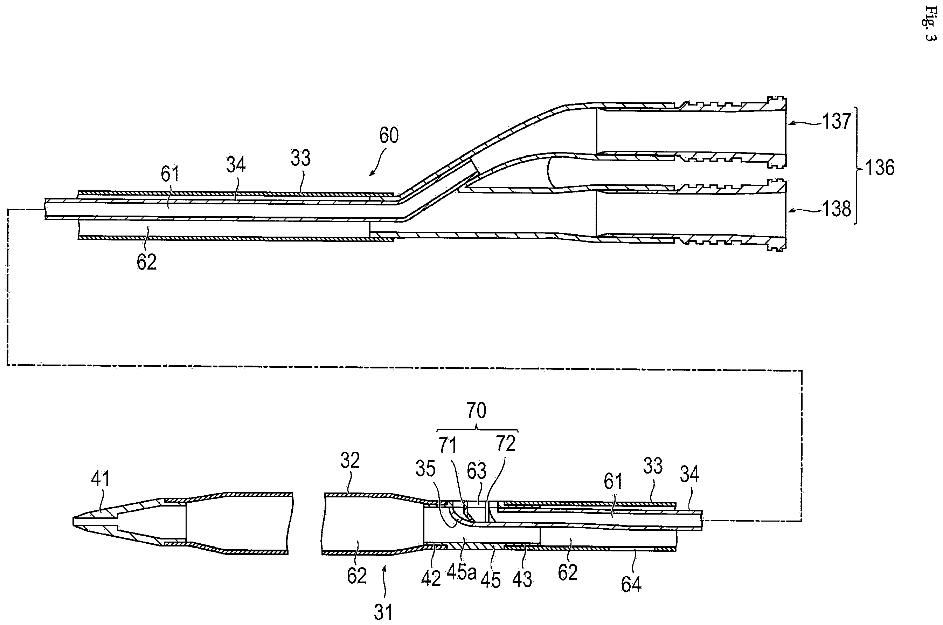

As illustrated in Fig. 3, the catheter 60 includes a first lumen 61 (corresponding to a blood feeding lumen) functioning as a blood feed path and extending in the X direction (corresponding to the axial direction), and a second lumen 62 (corresponding to a blood removing lumen) provided in parallel to the first lumen 61 and configured to function as a blood removal path. The first lumen 61 is formed within the third tube 34. The second lumen 62 is formed within the first tube 32, the second tube 33, and the connector 45, and extends from the distal end to the proximal end.

For inserting the catheter 60 in a living body, the dilator 50 illustrated in Fig. 2 is used. The dilator 50 is inserted through the second lumen 62 of the catheter 60, and the catheter 60 and the dilator 50 integrated in advance are inserted into the living body. Note that a method of using the catheter 60 will be described later in detail.

Configurations of each part of the catheter 60 will be described below.

As illustrated in Fig. 2 and Fig. 3, the catheter tube 31 includes the first tube 32, the second tube 33 connected to the proximal side of the first tube 32 via the connector 45, and the third tube 34 disposed in a lumen of the second tube 33.

The first tube 32 is configured to have a higher elasticity than the second tube 33. The first tube 32 is configured to have an outer diameter and an inner diameter larger than those of the second tube 33.

As illustrated in Fig. 3, the second tube 33 includes a blood removing side hole 64 (corresponding to "blood removing hole") that communicates with the second lumen 62, which corresponds to the blood removal path. The blood removing side hole 64 is formed into an oval shape.

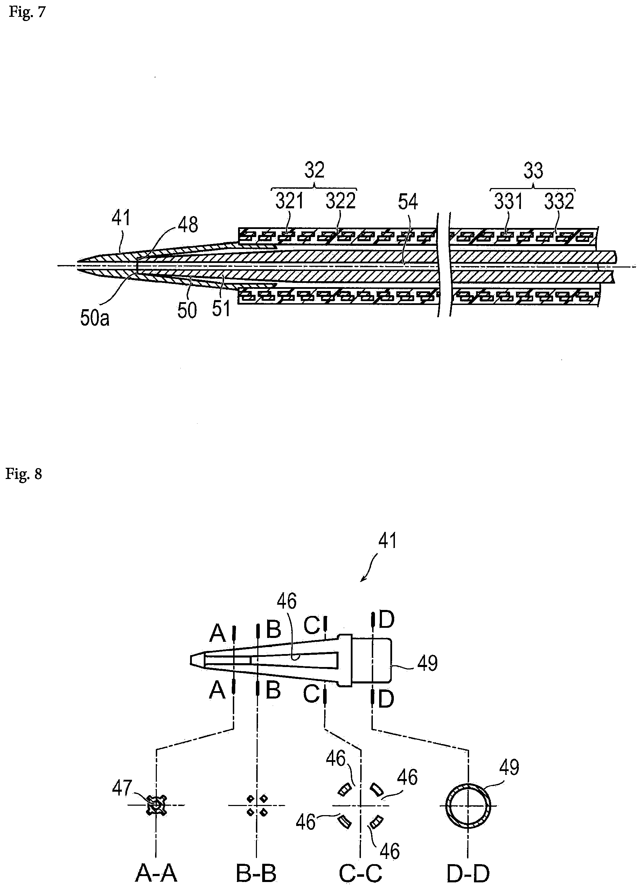

The first tube 32 and the second tube 33 each have a length necessary to dispose through-holes 46 and 47 (corresponding to the blood removing hole) of the distal tip 41 (see Fig. 8) and the blood removing side hole 64 of the second tube 33 at the position of the blood removal target. The length of the first tube 32 may be, for example, ranged from 20 cm to 40 cm, and the length of the second tube 33 may be, for example, ranged from 20 cm to 30 cm.

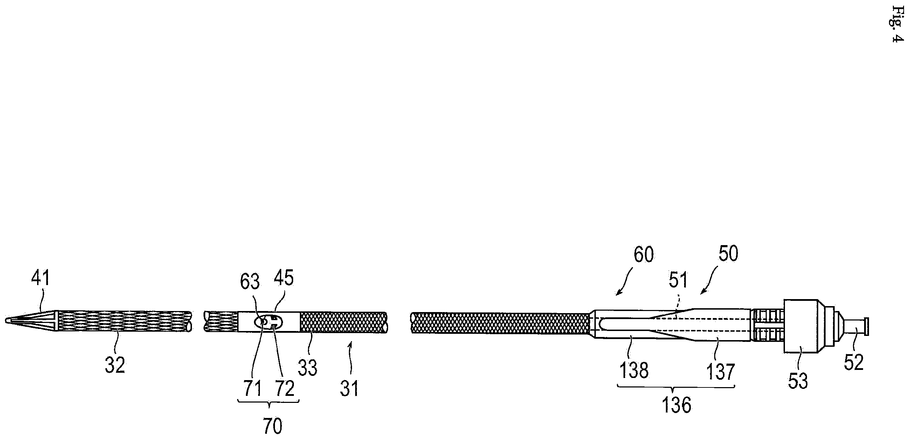

As illustrated in Fig. 4, the catheter 60 is inserted into a living body and indwelled so that the through-holes 46 and 47 of the distal tip 41 are placed in the inferior vena cava and the blood removing side hole 64 of the second tube 33 is placed in the internal jugular vein in a state in which the dilator 50 is inserted.

In a state in which the through-holes 46 and 47 of the distal tip 41 and the blood removing side hole 64 of the second tube 33 are disposed at a position of the target of blood removal, the first tube 32 is disposed in the inferior vena cava, which is a relatively thick blood vessel, and the second tube 33 is placed in a thigh vein, which is a relatively thin blood vessel.

When the dilator 50 is inserted into the second lumen 62 of the catheter 60, the first tube 32 having a high elasticity stretches in the axial direction as illustrated in Fig. 4, and thus the outer diameter and the inner diameter are reduced. At this time, the outer diameter of the first tube 32 becomes substantially the same as the outer diameter of the second tube 33. Since the catheter 60 is inserted into the living body in a state in which the first tube 32 is stretched in the axial direction and thus the outer diameter and the inner diameter are reduced, minimally invasive insertion of the catheter 60 is achieved.

In addition, when the dilator 50 is removed from the second lumen 62 of the catheter 60 after the catheter 60 is indwelled in the living body, the first tube 32 is contracted from the axially stretched state and thus the inner diameter of the first tube 32 increases. The first tube 32 here is placed in the inferior vena cava, which is a relatively thick blood vessel. Therefore, the outer diameter of the first tube 32 may be increased, and in association with this, the inner diameter of the first tube 32 may also be increased.

Pressure losses in the first tube 32 in this case are obtained respectively by multiplying the entire length of the first tube 32 by (average) cross-sectional area of the passage. In other words, by increasing the inner diameter of the first tube 32, the pressure loss in the first tube 32 is reduced. When the pressure loss in the first tube 32 is reduced, the flow rate of blood flowing in the circulation circuit increases. Therefore, in order to obtain a sufficient amount of circulation of blood, the inner diameter of the first tube 32 needs to be increased.

In contrast, when the thickness is substantially the same, if the inner diameters of the first tube 32 and the second tube 33 are increased, the outer diameters are increased correspondingly. Therefore, when the catheter 60 is inserted into the living body, a burden on the patient is increased, which impairs the minimally invasive method.

From the viewpoints described above, the inner diameter of the first tube 32 may be, for example, ranged from 9 mm to 11 mm, and the inner diameter of the second tube 33 may be, for example, ranged from 4 mm to 8 mm. In addition, the thickness of the first tube 32 and the second tube 33 may be ranged, for example, from 0.4 mm to 0.5 mm.

Also, as illustrated in Fig. 2, the first tube 32 preferably has tapered portion at the distal end portion and the proximal end portion. The tapered portion is gradually reduced in diameter from the center of the first tube 32 outward in the axial direction. Accordingly, the inner diameters of the distal end and the proximal end of the first tube 32 continue to the inner diameter of the distal tip 41 disposed on the distal side and to the inner diameter of the connector 45 disposed on the proximal side.

Configurations of the first tube 32 and the second tube 33 will be described further in detail below.

The first tube 32 includes a first reinforcement body 321 made of a wire W braided so as to intersect, and a first resin layer 322 provided so as to cover the first reinforcement body 321 as illustrated in Fig. 7.

The second tube 33 includes a second reinforcement body 331 made of a wire W braided so as to intersect, and a second resin layer 332 provided so as to cover the second reinforcement body 331 as illustrated in Fig. 7.

In the present embodiment, the wire W is made of a shape memory material such as a shape memory metal or a shape memory resin which are publicly known. The shape memory metal that may be used here includes, for example, titanium-based (e.g., Ni-Ti, Ti-Pd, Ti-Nb-Sn) or copper-based alloy. The shape memory resin includes, for example, acrylic resin, transisoprene polymer, polynorbornene, styrene-butadiene copolymer, and polyurethane.

Since the wire W is made of the shape memory material, a contracting distance of the first tube 32 along the axial direction occurring in association with removal of the dilator 50 from the catheter 60 is the same as the stretching distance of the first tube 32 along the axial direction occurring for inserting the dilator 50 into the catheter 60.

The first reinforcement body 321 of the first tube 32 is braided so as to be coarser than the second reinforcement body 331 of the second tube 33 as illustrated in Fig. 2. According to this configuration, the first tube 32 may be made more flexible than the second tube 33, and the elasticity may be enhanced as well.

The line diameter of the wire W preferably ranges from 0.1 mm to 0.2 mm.

The first resin layer 322 of the first tube 32 is made of a soft material having lower hardness than the second resin layer 332 of the second tube 33. According to this configuration, the first tube 32 may be made more flexible than the second tube 33, and the elasticity may be enhanced as well.

The first and second resin layers 322 and 332 may be made of vinyl chloride, silicone, polyethylene, nylon, urethane, polyurethane, fluororesin, thermoplastic elastomer resin, or the like, or may be made using a composite material thereof.

The silicone materials have high biocompatibility and are soft by themselves. Therefore, the silicone materials have a characteristic that they can hardly damage the blood vessel. The polyethylene materials are soft and have a hardness that is resistant to pressure. Moreover, the polyethylene materials have biocompatibility comparable to that of the silicone materials. The polyethylene materials are harder than silicone and have a characteristic that they are easy to insert into thin blood vessels. The polyurethane materials have a property of softening after insertion. Taking advantage of these properties, these materials may be used as the materials for the first and second resin layers 322 and 332.

Hydrophilic coating may be applied to the polyurethane material. In this case, the tube, having a smooth surface, is easily inserted into blood vessel and is less likely to damage the blood vessel wall. Probability of adhesion of blood or protein is low, and prevention of formation of thrombus is expected.

The method of forming the tubes 32, 33 are not specifically limited, but may be formed by, for example, dip coating (immersion method) or insert molding. Note that the reinforcement bodies 321 and 331 need only be covered over at least the outer surfaces thereof with the resin layers 322 and 332.

The third tube 34 is disposed in the lumen of the second tube 33 as illustrated in Fig. 3. The third tube 34 is inserted from the proximal side of the second tube 33 into the second lumen 62, and a distal end of the first lumen 61 communicates with a blood feeding side hole 63.

The distal end of the third tube 34 is provided with an inclined portion 35 inclined toward the blood feeding side hole 63.

The length of the third tube 34 is configured to be longer than the length of the second tube 33, and may be ranged, for example, from 15 cm to 25 cm. The cross-sectional area of the third tube 34 may be ranged, for example, from 11 mm² to 15 mm².

The third tube 34 may be made of vinyl chloride, silicone, polyethylene, nylon, urethane, polyurethane, fluororesin, thermoplastic elastomer resin, or the like, or may be made using a composite material thereof.

The connector 45 is a joint member connecting the first tube 32 and the second tube 33 as illustrated in Fig. 2 to Fig. 4. The connector 45 is a housing as a whole, and is made of, for example, a hard plastic.

The connector 45 includes connection sections 42 and 43, which are reduced diameter portions, at both end portions of the cylindrical member as illustrated in Fig. 3. A liquid passage 45a provided in an interior of the connector 45 communicates with the first and second tubes 32 and 33 by inserting the connection sections 42 and 43 into the first and second tubes 32 and 33.

The connector 45 includes the blood feeding side hole 63 opening on a side surface. The blood feeding side hole 63 is disposed at a position of a target of blood feeding in a living body, and the blood oxygenated by the oxygenator 2 is fed into the living body through the blood feeding side hole 63. The blood feeding side hole 63 is formed into an outer cylindrical surface of connector 45 as an oval shape with a distal end and a proximal end of the oval shape. Note that the cylindrical body of connector 45 is omitted for the sake of easy understanding in Fig. 6B.

The rectification part 70 is capable of dispersing blood flowing through the first lumen 61 when the blood is allowed to flow out from the blood feeding side hole 63 (see Fig. 6) . The rectification part 70 is disposed in the first lumen 61 at the position facing the blood feeding side hole 63 as illustrated in Fig. 2 to Fig. 6. In other words, the rectification part 70 is disposed at a position opposing the blood feeding side hole 63 when viewed from above.

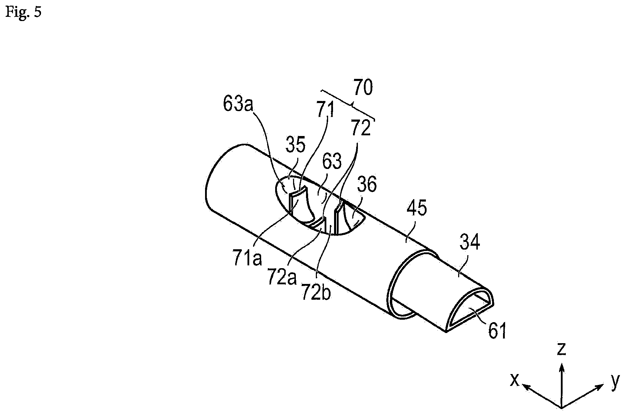

The rectification part 70 is configured to rise in the Z direction intersecting the X direction as illustrated in Fig. 5. The rectification part 70 includes a first projection 71 provided at a distal side in the X direction and second projections 72 provided at a proximal side of the first projection 71.

The first projection 71 is fixed to the inclined portion 35 of the third tube 34 as illustrated in Fig. 6B. A method of fixing the first projection 71 and the inclined portion 35 is not specifically limited, but may be fixed, for example, by adhesion with an adhesive agent. Note that the first projection 71 may be configured integrally with the third tube 34.

The first projection 71 is disposed at a position near the center in the Y direction at a position facing the blood feeding side hole 63 as illustrated in Fig. 5 and Fig. 6A.

The first projection 71 includes a guiding section 71a configured to guide blood flow in the first lumen 61 outward in the Y direction (a width direction of the blood flow) as illustrated in Fig. 6A. Specifically, the guiding section 71a has a curved shape curving outward in the Y direction toward the distal side.

The first projection 71 preferably rises gently so as to be capable of guiding the blood flow in the first lumen 61 smoothly when guiding the blood flow from the X direction to the Z direction as illustrated in Fig. 6B.

The second projections 72 are fixed to a linear section 36 of the third tube 34 on the proximal side of the first projection 71 as illustrated in Fig. 5 and Fig. 6B. A method of fixing the second projections 72 and the linear section 36 is not specifically limited, but may be fixed, for example, by adhesion with an adhesive agent. Note that the second projections 72 may be formed integrally with the third tube 34.

A pair of the second projections 72 are provided at the same position in the X direction and at different positions when viewed in the X direction as illustrated in Fig. 6A. In other words, the pair of second projections 72 are provided at the different positions in the Y direction. A gap 72b opposing the first projection 71 in the X direction is formed between the pair of second projections 72.

In this manner, the first projection 71 is disposed at a position different from the second projections 72 when viewed in the X direction.

The pair of second projections 72 each include a guiding section 72a guiding the blood flow outward in the Y direction. Specifically, the guiding sections 72a may be formed into a curved shape curving as it goes outward in the Y direction.

The second projections 72 preferably rise gently so as to be capable of guiding the blood flow in the first lumen 61 smoothly when guiding the blood flow from the X direction to the Z direction as illustrated in Fig. 6B.

The distal tip 41 is disposed at a distal end of the first tube 32 as illustrated in Fig. 7. The distal tip 41 is provided with a tapered shape decreasing in diameter as it goes toward the distal side.

A flat receiving surface 48 that is attached on a flat surface 50a of the dilator 50 used prior to the insertion of the catheter 60 into the living body is formed inside the distal tip 41.

The distal tip 41 includes a base portion 49 to be inserted to the distal end of the first tube 32, a plurality of the through-holes 46 provided on a side surface, and the through-hole 47 provided at a distal end of the distal tip 41 as illustrated in Fig. 8. The through-holes 46 and 47 function as holes for blood removal. The through-hole 47 in the distal tip 41 constitutes a part of the second lumen 62 of the catheter 60. The distal tip 41 may be made, for example, of hard plastic.

The through-holes 46 and 47 formed in the distal tip 41 and the blood removing side hole 64 formed in the second tube 33 are arranged at positions of different targets of blood removal in the living body, so that efficient blood removal is achieved. Even when the through-holes 46 and 47 or the blood removing side hole 64 are blocked by being adsorbed on the blood vessel wall, blood removal is possible through the hole which is not blocked, so that stable extracorporeal circulation is achieved.

By fixing the hard distal tip 41 to the distal end portion of the first tube 32, the first tube 32 is effectively prevented from being collapsed during blood removal.

The lock connector 136 includes a first lock connector 137 communicating with the first lumen 61 and a second lock connector 138 provided in parallel to the first lock connector 137 and communicating with the second lumen 62 as illustrated in Fig. 3. The lock connector 136 is a Y-shaped Y connector formed of the first lock connector 137 bifurcated from the second lock connector 138.

The first lock connector 137 is connected to the proximal portion of the third tube 34. The second lock connector 138 is coaxially connected to the proximal portion of the second tube 33. The blood feed tube 12 (see Fig. 1) is connected to the first lock connector 137, and the blood removal tube 11 (see Fig. 1) is connected to the second lock connector 138.

Next, the configuration of the dilator 50 will be described.

The dilator 50 includes a dilator tube 51 provided so as to extend in the axial direction, a dilator hub 52 to which the proximal end of the dilator tube 51 is fixed, and a screw ring 53 provided at a distal end of the dilator hub 52.

The dilator tube 51 is an elongated body extending in the axial direction and having a relatively high rigidity. The entire length of the dilator tube 51 along the axial direction is longer than the entire length of the catheter 60 along the axial direction. The dilator tube 51 includes a guide wire lumen 54 which allows an insertion of the guide wire (not illustrated) . The dilator tube 51 is guided by the guide wire and is inserted into the living body together with the catheter 60. The dilator tube 51 is removed from the catheter 60 by pulling the dilator hub 52 toward the proximal side after the catheter 60 is indwelled in the living body.

The distal end of the dilator tube 51 is provided with the flat surface 50a on which the receiving surface 48 of the distal tip 41 is attached as illustrated in Fig. 7. The dilator tube 51 has a relatively high rigidity and is firm enough to allow a pushing force toward the distal side by operation at hand to be transmitted to the distal tip 41. Therefore, the dilator tube 51 achieves a role to widen a narrow blood vessel by bringing the flat surface 50a thereof into attachment with the receiving surface 48 of the distal tip 41 and pushing the distal tip 41 toward the distal side.

The screw ring 53 includes a female screw section (not illustrated) provided with a screw groove on an inner surface of the lumen. The dilator 50 is configured to be mounted on the catheter 60 by screwing the female screw section of the screw ring 53 into a male screw section 138A of the second lock connector 138.

### Method of Using Catheter

Next, a method of using the catheter 60 described above will be described. Fig. 2 illustrates a state before the dilator tube 51 of the dilator 50 is inserted into the second lumen 62 in the catheter 60, and Fig. 4 illustrates a state after the dilator tube 51 is inserted through the second lumen 62 in the catheter 60.

First, as illustrated in Fig. 4, the dilator tube 51 of the dilator 50 is inserted into the second lumen 62 in the catheter 60. The dilator tube 51 passes through interiors of the second tube 33 and the first tube 32 in sequence, and the flat surface 50a of the dilator tube 51 is attached on the receiving surface 48 of the distal tip 41.

The entire length of the dilator tube 51 in the axial direction here is longer than the entire length of the catheter 60 in the axial direction. Therefore, the distal tip 41 is pressed toward the distal side in a state in which the flat surface 50a of the dilator tube 51 is attached on the receiving surface 48 of the distal tip 41. Accordingly, the distal end of the first tube 32 fixed to the distal tip 41 is pulled toward the distal side. Accordingly, the catheter 60 receives a stretching force in the axial direction, and the first tube 32, which has relatively high elasticity in the catheter 60, stretches in the axial direction. Subsequently, the proximal end of the catheter 60 is fixed to the dilator hub 52.

At this time, the first tube 32 is changed from a state illustrated in Fig. 2 to a state illustrated in Fig. 4. In other words, the first tube 32 stretches in the axial direction and, simultaneously, the outer diameter of the first tube 32 is reduced and becomes substantially the same as the outer diameter of the second tube 33.

Next, the catheter 60 in which the dilator 50 is inserted is inserted along a guide wire (not illustrated) which is inserted to a target portion in the living body in advance. Since the dilator 50 is inserted into the catheter 60 at this time, the outer diameter of the first tube 32 is substantially the same as the outer diameter of the second tube 33. Therefore, minimally invasive insertion of the catheter 60 into the living body is achieved, and thus the burden on the patient body may be suppressed.

The catheter 60 is inserted into a living body and indwelled until the through-holes 46 and 47 of the distal tip 41 are placed in the inferior vena cava, the blood removing side hole 64 of the second tube 33 is placed in the internal jugular vein, and the blood feeding side hole 63 of the connector 45 is placed in the right atrium. In this state, the first tube 32 is disposed in the inferior vena cava, which is a relatively thick blood vessel, and the second tube 33 is placed in a thigh vein, which is a relatively thin blood vessel.

Next, the dilator tube 51 and the guide wire are removed from the catheter 60. By removing the dilator tube 51 from the catheter 60, the catheter 60 is released from a stretching force in the axial direction that the catheter 60 receives from the dilator 50. Therefore, the first tube 32 is contracted in the axial direction, and the inner diameter of the first tube 32 is increased. Accordingly, the pressure loss in the first tube 32 is reduced and thus a required flow rate of liquid is secured.

Next, the first lock connector 137 is connected to the blood feed tube 12 of the extracorporeal circulator 1 in Fig. 1, and the second lock connector 138 is connected to the blood removal tube 11 of the extracorporeal circulator 1 in Fig. 1. Subsequently, extracorporeal circulation is started.

During the extracorporeal circulation, blood flows out from the blood feeding side hole 63.

The effect of the catheter 60 according to the present embodiment will now be described while explaining a flow in the vicinity of the rectification part 70 of blood B flowing in the first lumen 61.

Referring first to Fig. 6A, the flow of the blood B when viewing the catheter 60 in the Z direction will be described.

Part of the blood B flowing through the first lumen 61, that is, blood B1 flowing in an outer part in the Y direction collides with the pair of second projections 72 and flows along the guiding sections 72a of the second projections 72, and is allowed to flow out from the blood feeding side hole 63 outward in the Y direction.

Part of the blood B flowing through the first lumen 61, that is, blood B2 flowing near a center in the Y direction passes through the gap 72b between the second projections 72, then flows toward the distal side, and collides with the first projection 71. The blood B2 which collides with the first projection 71 flows along the guiding section 71a of the first projection 71, and flows outward in the Y direction from the blood feeding side hole 63.

Part of the blood B2, that is, blood B3 flowing around the first projection 71 flows along the inclined portion 35 and flows out from a distal end 63a of the blood feeding side hole 63.

Next, referring to Fig. 6B, a flow of the blood B when viewing the catheter 60 in the Y direction will be described. Note that the connector 45 is omitted for the sake of easy understanding in Fig. 6B.

Part of the blood B flowing through the first lumen 61, that is, the blood B1 flowing in an outer part in the Y direction collides with the pair of second projections 72, and is allowed to flow out from the blood feeding side hole 63 upward in the Z direction.

Part of the blood B flowing through the first lumen 61, that is, the blood B2 flowing near a center in the Y direction passes through the gap 72b between the second projections 72, then flows toward the distal side, and collides with the first projection 71. The blood B2 colliding with the first projection 71 flows out upward in the Z direction from the blood feeding side hole 63. Since the first projection 71 is fixed to the inclined portion 35 and raises gently, the blood B2 can be fed smoothly out from the blood feeding side hole 63.

Part of the blood B2, that is, the blood B3 flowing around the first projection 71 flows along the inclined portion 35 and flows out in a direction tilted from the Z direction toward the distal side from the distal end 63a of the blood feeding side hole 63

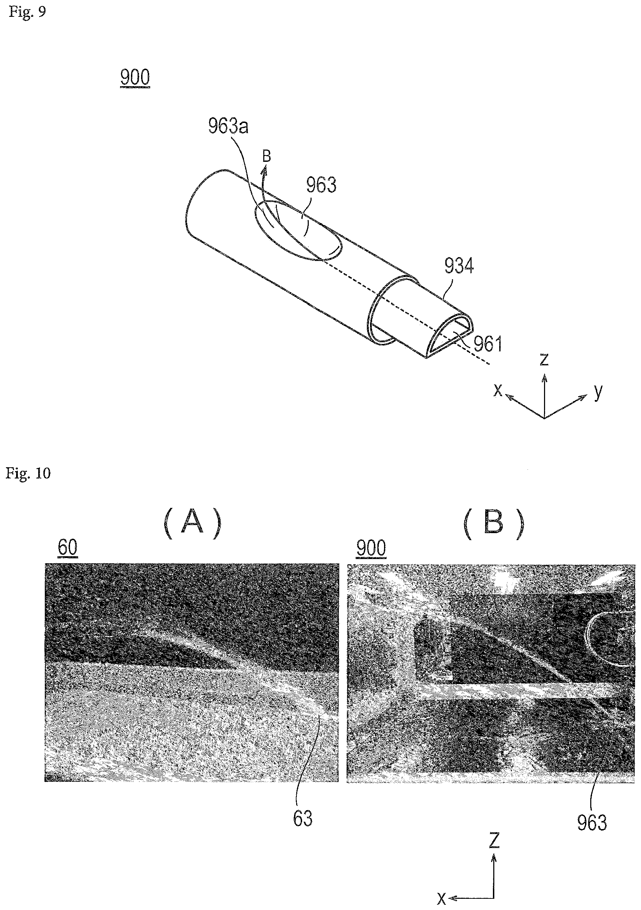

For example, if the rectification part is not provided in a catheter 900 as illustrated in Fig. 9, the blood B flowing through a first lumen 961 of a third tube 934 flows out locally from a distal end 963a of a blood feeding side hole 963, so that the blood may strongly collide with the blood vessel wall.

In contrast, since the catheter 60 according to the present embodiment includes the rectification part 70 as described above, the blood B flowing through the first lumen 61 is dispersed into the blood B1 which collides with the second projections 72 and flows out, into the blood B2 which collides with the first projection 71 and flows out, and into the blood B3 which flows out along the inclined portion 35, as illustrated in Figs. 6A and 6B. The blood B flowing through the first lumen 61 is allowed to flow out outward in the Y direction by the guiding section 71a of the first projection 71 and the guiding sections 72a of the second projections 72 as illustrated in Fig. 6A. Therefore, according to the catheter 60 of the present embodiment, the blood flowing out from the blood feeding side hole 63 is dispersed so that the collision of the blood with the blood vessel wall is softened, compared with the catheter 900 according to the comparative example.

When the extracorporeal circulation is terminated, the catheter 60 is removed from the blood vessel, and hemostasis is performed on an insertion point by a surgical procedure as required.

Referring now to Fig. 10 to Fig. 12, a result of visual observation when water is allowed to flow out from the blood feeding side holes 63 and 963 of the catheter 60 of the embodiment and the catheter 900 according to the comparative example, respectively.

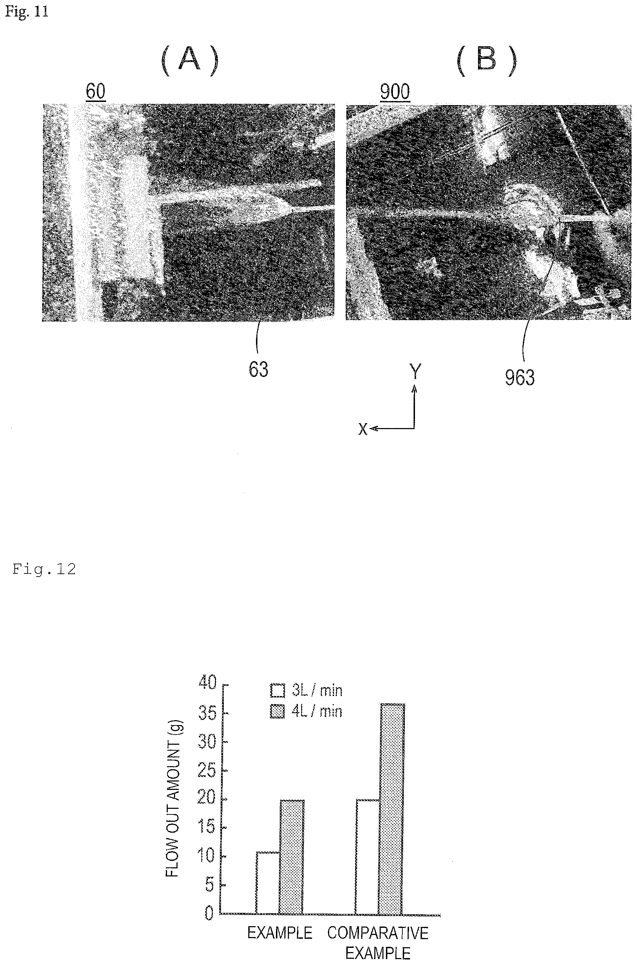

Fig. 10 shows a state in which water flows out when viewing the catheters 60 and 900 in the Y direction, in which Fig. 10A shows a state in which water flows out from the blood feeding side hole 63 of the catheter 60 according to the present embodiment, and Fig. 10B shows a state in which water is allowed to flow out from the blood feeding side hole 963 of the catheter 900 according to the comparative example. Fig. 11 shows a state in which water flows out when viewing the catheters in the Z direction, in which Fig. 11A shows a state in which water flows out from the blood feeding side hole 63 of the catheter 60 according to the present embodiment, and Fig. 11B shows a state in which water is allowed to flow out from the blood feeding side hole 963 of the catheter 900 according to the comparative example.

As a result of the visual observation, it is apparent that the flow is dispersed in the X direction and the Z direction in the case of the catheter 60 of the embodiment illustrated in Fig. 10A in contrast to the catheter 900 according to the comparative example illustrated in Fig. 10B. This is because the blood B flowing in the first lumen 61 is dispersed into the blood B1 which collides with the first projection 71 and flows out, the blood B2 which collides with the second projections 72 and flows out, and the blood B3 which flows out from the inclined portion 35.

It is also seen that the flow is dispersed along the Y direction in the case of the catheter 60 of the embodiment illustrated in Fig. 11A compared with the catheter 900 according to the comparative example illustrated in Fig. 11B. This is because the blood B flowing in the first lumen 61 is guided outward in the Y direction by the guiding section 71a provided on the first projection 71 and the guiding sections 72a provided on the second projections 72.

In addition to the visual observation, scales were provided at positions 3 cm apart respectively from the blood feeding side hole 63 of the catheter 60 according to the embodiment and the blood feeding side hole 963 of the catheter 900 according to the comparative example and load values flowing out from the blood feeding side holes 63 and 963 were measured. Specifically, in order to simulate the interior of the blood vessel, the catheters 60 and 900 were immersed, and load values of water flowing from the blood feeding side holes 63 and 963 were measured under the condition of a flow rate of 4 L/min. or 3 L/min. The result of measurement is shown in Fig. 12.

As illustrated in Fig. 12, when the flow rate is 4 L/min. , the load value of water flowing out from the blood feeding side hole 63 of the catheter 60 according to the embodiment was 20 g, and the load value of water flowing out from the blood feeding side hole 963 of the catheter 900 according to the comparative example was 37 g.

Likewise, when the flow rate was 3 L/min. , the load value of water flowing out from the blood feeding side hole 63 of the catheter 60 according to the embodiment was 11 g, and the load value of water flowing out from the blood feeding side hole 963 of the catheter 900 according to the comparative example was 20 g.

From the result described above, it was found that the load value of water flowing out from the blood feeding side hole 63 of the catheter 60 was lowered by the provision of the rectification part 70 in the catheter 60. Therefore, the rectification part 70 provided in the catheter 60 can soften the collision of the blood with the blood vessel wall.

As described above, the catheter 60 according to the present embodiment is the catheter 60 extending in the X direction and allowing blood to pass therethrough. The catheter 60 includes the first lumen 61 extending in the axial direction, the blood feeding side hole 63 communicating with the distal end of the first lumen 61, and the rectification part 70 disposed in the first lumen 61 at a position facing the blood feeding side hole 63 and rising in the direction intersecting with the X direction.

According to the catheter 60 configured in this manner, since the rectification part 70 is provided on the position facing the blood feeding side hole 63, blood passing through the first lumen 61 collides the rectification part 70, and thus blood flowing out from the catheter 60 can be dispersed. Therefore, the collision of blood with the blood vessel wall can be softened.

In addition, the rectification part 70 includes the first projection 71 and the second projections 72 provided at the proximal side of the first projection 71 in the X direction. The first projection 71 is disposed at the positions different from the second projections 72 when viewed in the X direction. According to the catheter 60 configured in this manner, part of the blood flowing through the first lumen 61 and passing between the second projections 72 collides with the first projection 71. Therefore, the blood can be allowed to flow out from the blood feeding side hole 63 at different positions in the X direction, and the blood flowing out from the catheter 60 can be dispersed so that the collision of the blood with the blood vessel wall can be softened.

Also, the pair of second projections 72 are provided at the same position in the X direction and at different positions when viewed in the X direction, and the gap 72b opposing the first projection 71 in the X direction is formed between the pair of second projections 72. According to the catheter 60 configured in this manner, part of the blood flowing through the first lumen 61 and passing through the gap 72b between the second projections 72 collides with the first projection 71. Therefore, the blood can be allowed to flow out from the blood feeding side hole 63 at different positions in the X direction, and the blood flowing out from the catheter 60 can be dispersed so that the collision of the blood with the blood vessel wall can be softened.

In addition, the catheter 60 further includes an inclined portion 35 inclined from the first lumen 61 toward the blood feeding side hole 63, and the first projection 71 is disposed on the inclined portion 35. According to the catheter 60 configured in this manner, part of the blood B flowing through the first lumen 61, that is, the blood B2 that collides with the first projection 71 can be allowed to flow out smoothly from the blood feeding side hole 63.

The rectification part 70 includes the guiding sections 71a and 72a configured to guide the blood flow in the first lumen 61 outward in the Y direction. According to the catheter 60 configured in this manner, the blood B flowing through the first lumen 61 is allowed to flow out outward in the Y direction by the guiding section 71a of the first projection 71 and the guiding sections 72a of the second projections 72 as illustrated in Fig. 6A. Therefore, the blood flowing out from the blood feeding side hole 63 can be dispersed and thus the collision of the blood with the blood vessel wall can be softened.

Further, the second lumen 62 provided in parallel with the first lumen 61, the through-holes 46 and 47 communicating with the second lumen 62, and the blood removing side hole 64 are further provided. According to the catheter 60 configured in this manner, both the blood removal and the blood feed are achieved in the single catheters 60.

Although the catheter 60 according to the present invention has been described through the embodiments thus far, the present invention is not limited to the configuration described in the embodiments and the modified example, and may be modified as needed based on the description of the Claims.

For example, in the above-described embodiment, the rectification part 70 includes the first projection 71 and the second projections 72. However, the configuration of the rectification part 70 is not particularly limited as long as it has a shape that rises in a direction intersecting with the X direction.

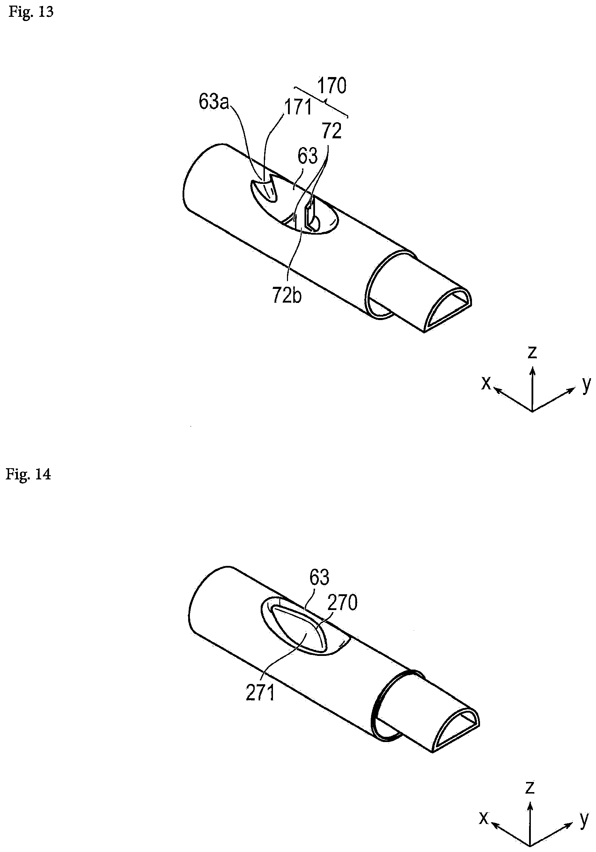

For example, as illustrated in Fig. 13, a rectification part 170 has a first projection 171 provided on the distal side and the second projections 72, and the first projection 171 may be formed on the distal end 63a of the blood feeding side hole 63.

Also, as illustrated in Fig. 14, a rectification part 270 may include a single projection 271, and the projection 271 may have a streamline shape. According to the rectification part 270 configured in this manner, the projection 271 is formed in a streamline shape, so that the stagnation of the blood can be suitably prevented.

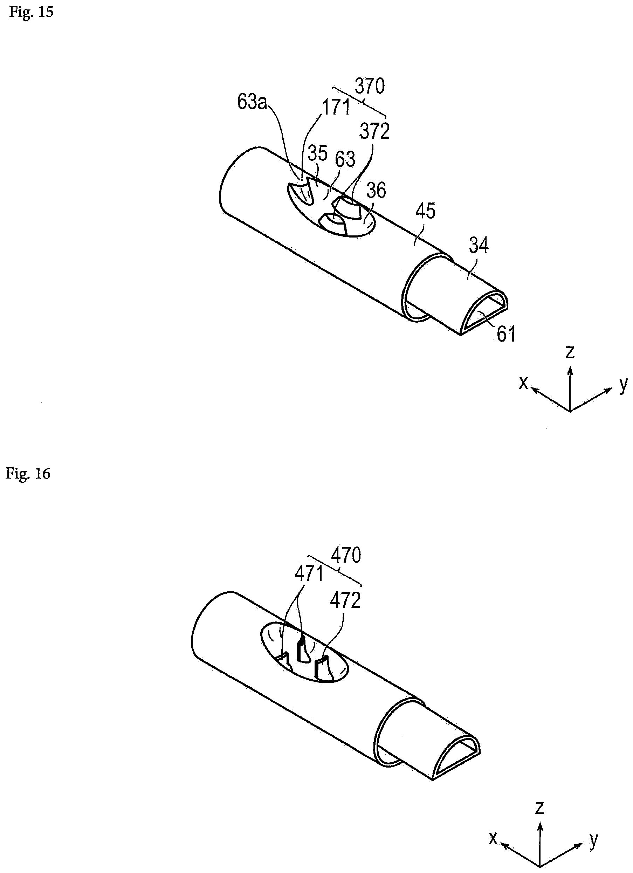

Also, as illustrated in Fig. 15, a rectification part 370 may include the first projection 171 and second projections 372, and the second projections 372 may have a streamline shape in the same manner as the projection 271. According to the rectification part 370 configured in this manner, the second projections 372 are formed in a streamline shape, so that the stagnation of the blood can be suitably prevented.

In the above-described embodiment, the single first projection 71 is provided on the distal side, and two of the second projections 72 are provided on the proximal side. However, as illustrated in Fig. 16, a rectification part 470 may include two first projections 471 on the distal side, and one second projection 472 may be provided on the proximal side.

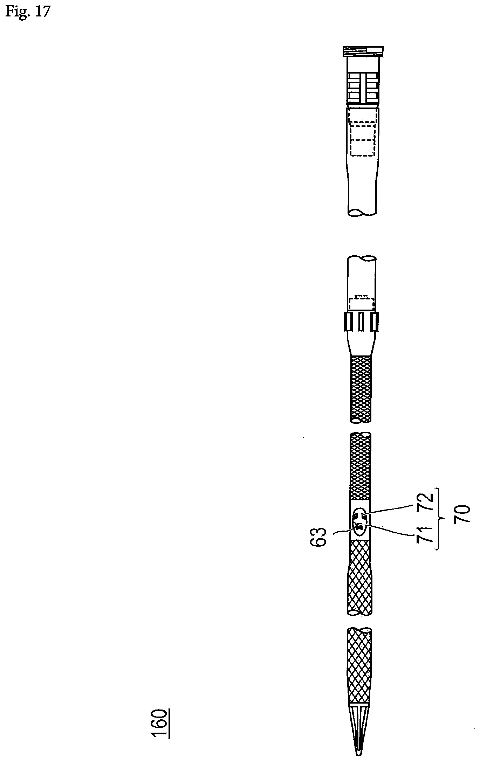

In the above-described embodiment, the catheter 60 is used as a double lumen catheter. However, as illustrated in Fig. 17, a catheter 160 may be used as a blood feeding catheter having a single lumen.

Also, in the above-described embodiment, the rectification part 70 is configured to rise in the Z direction intersecting the X direction. However, the rectification part 70 may be configured to rise in the Y direction intersecting the X direction.

Further, the material constituting the wire W is not limited to the configuration of shape memory material as long as the material has a restoring force, which is a force returning to its original shape after deformation, and has a function to reinforce the resin layer, and may be made of, for example, a known resilient material.

This application is based on Japanese Patent Application No. 2016-244565 filed on December 16, 2016, the disclosure of which is incorporated herein by reference in its entirety.

### Reference Signs List

- 35: inclined portion
- 46, 47: through-hole (blood removing hole)
- 60, 160: catheter
- 61: first lumen (blood feeding lumen)
- 61a: distal end of first lumen
- 62: second lumen (blood removing lumen)
- 63: blood feeding side hole
- 64: blood removing side hole (blood removing hole)
- 70, 170, 270, 370, 470: rectification part
- 71, 171, 471: first projection
- 271: projection
- 71a: guiding section
- 72, 372, 472: second projection
- 72a: guiding section
- 72b: gap
- X: axial direction
- Y: width direction of blood flow

## Claims

1. A catheter extending in an axial direction for allowing passage of blood, comprising:
a blood feeding lumen extending in the axial direction;
a blood feeding side hole communicating with a distal end of the blood feeding lumen; and
a rectification part that is disposed in the blood feeding lumen at a position facing the blood feeding side hole and rises in a direction intersecting with the axial direction.

2. The catheter according to claim 1, wherein
the rectification part including:
a first projection; and
a second projection provided on a proximal side of the first projection in the axial direction, wherein
the first projection is provided at a position different from the second projection when viewed in the axial direction.

3. The catheter according to claim 2, wherein
a pair of the second projections are provided at an identical position in the axial direction and at different positions when viewed in the axial direction, and
a gap is formed between the pair of second projections at a position opposing the first projection in the axial direction.

4. The catheter according to any one of claims 1 to 3, further comprising an inclined portion inclined from the blood feeding lumen toward the blood feeding side hole, wherein
the rectification part is disposed on the inclined portion.

5. The catheter according to any one of claims 1 to 4, wherein the rectification part includes a guiding section configured to guide the blood flow in the blood feeding lumen outward in a width direction of the blood flow.

6. The catheter according to any one of claims 1 to 5, further comprising:
a blood removing lumen provided in parallel with the blood feeding lumen; and
a blood removing hole communicating with the blood removing lumen.
